# EUROPEAN PATENT APPLICATION

(11) **EP 2 749 261 A1**
(43) Date of publication of application: **02.07.2014**
(21) Application number: 13198482.5
(22) Date of filing: 19.12.2013
(51) Int. Cl.: A61F 13/20, A61F 13/34

(54) **Catamenial Tampon**

(30) Priority: 28.12.2012 JP 2012288935
(71) Applicant: Unicharm Corporation, Ehime-Ken Ehime (JP)
(72) Inventor: Yamaki, Koichi, Kanonji-shi, Kagawa (JP)
(74) Representative: Eke, Philippa Dianne

(57) **Abstract**

A catamenial tampon (1) includes a tampon body (2) and a withdrawal string (3). The tampon body (2) is composed of an absorbent material (12) formed of a liquid-absorptive sponge and a wrapping material (13) formed of a liquid-permeable sheet material and adapted to wrap the absorbent material (12). The withdrawal string (3) is attached to the wrapping material (13).

## Description

### {Technical Field}

The present invention relates to catamenial tampons.

### {Background}

Conventionally, catamenial tampons are known. For example, the catamenial tampon disclosed in JP 1982-160458 A (Patent Literature 1) includes a tampon body and a withdrawal string wherein the tampon body is formed of a strip of layered cotton fibers cylindrically rolled up so as to have spiral-patterned end surfaces and then diametrically compressed. The tampon body is provided with the withdrawal string for taking out the inserted tampon body from the wearer's vaginal cavity.

JP 1991-109067 A (Patent Literature 2) discloses a compressed cellulose sponge particularly suitable to be used as an absorbent body in absorbent articles such as the catamenial napkin. The term used herein "cellulose sponge" means the sponge formed of the material having a cellulose skeletal structure. As such sponge, not only the sponge formed of cellulose itself, sponges formed of cellulose derivatives,for example,viscose,cellulose ethers, cellulose esters or a mixture thereof are included.

JP 2007-105217 A (Patent Literature 3) discloses an invention relating to an absorbent article and a method of producing the same. The absorbent article described herein is provided with an absorbent body formed of a hydrophilic foam. The hydrophilic foam is used in a state containing water and/or polyhydric alcohol in a range of 60 to 600 % by mass. As the hydrophilic foam, in addition to the cellulose sponge described in Patent Literature 2, a hydrophilic porous sheet obtained by layering hydrophilic porous fibers and compressing them, a hydrophilized urethane foam, a hydrophilized ethylene-vinyl acetate copolymer or hydrophilized polyolefin or a melamine resin foam may be used.

JP 2007-197649 A (Patent Literature 4) discloses a flexible sponge formed of polysaccharide substance and suitable for a constituent of the catamenial tampon and the diaper. This sponge is produced by following the steps of mixing a water-soluble polymer into viscose solution or cuprammonium solution or organic solvent solution of polysaccharide, heat-treating the mixture after the solution has been solidified, and freeze-drying the mixture. As polysaccharide, cellulose or derivatives thereof, chitin or deacetylated chitin or derivatives thereof, a mixture of cellulose and chitin or a mixture of cellulose and deacetylated chitin may be used.

### {Citation List}

### {Patent Literature}

{PTL 1}: JP 1982-160458 A
{PTL 2}: JP 1991-109067 A
{PTL 3}: JP 2007-105217 A
{PTL 4}: JP 2007-197649 A

### {Summary}

### {Technical Problem}

Among various types of catamenial tampons, the tampon formed of compressed layered cotton fibers swells upon absorption of bodily fluid after having been inserted into the vaginal cavity and sometimes presses against the vaginal wall, whereby the wearer of the catamenial tampon might feel a sense of discomfort.

The sponge formed from polysaccharide such as cellulose or derivatives thereof should not remarkably swell due to absorption of the body exudates when such sponge is used as the tampon body of the catamenial tampon. However, such sponges generally have a low resistance to external force such as tensile force, tearing force, bending force and twisting force, and it is difficult to draw out the used tampon body formed of such sponge smoothly from the vaginal cavity with use of the withdrawal string attached to the tampon body.

An object of the present invention is to provide a catamenial tampon having a tampon body formed of a liquid-absorptive sponge adapted to be smoothly drawn out from the vaginal cavity with use of the withdrawal string attached to the tampon body.

### {Solution to Problem}

Some embodiments of the present invention provide a catamenial tampon including a tampon body adapted to be inserted into the vaginal cavity and a withdrawal string used to draw out the tampon body from the vaginal cavity.

In this catamenial tampon, the tampon body includes an absorbent material formed from a liquid-absorptive sponge and a wrapping material formed of a liquid-permeable sheet material and adapted to wrap the absorbent material, and the withdrawal string is attached to the sheet material so as to extend in a direction in which the tampon body is drawn out from the vaginal cavity.

According to an embodiment of the present invention, the sheet material may be selected from a nonwoven fabric formed of thermoplastic synthetic fibers, a perforated film formed from a thermoplastic synthetic resin and a net formed from thermoplastic synthetic resin.

According to another embodiment of the present invention, the absorbent material is formed of planar absorbent sponge and the withdrawal string is doubled up so as to pinch the planar absorbent sponge in a thickness direction thereof, and the sponge is spirally rolled up together with the sheet material to form a roll so that the withdrawal string may extend from a diametrical center of the roll in an axial direction of the roll.

According to still another embodiment of the present invention, the absorbent material is formed of a columnar sponge and contained within a sac formed of the sheet material, and the withdrawal string is attached to a periphery of an opening defined by the sac so that the withdrawal string may be pulled against the sac to close the opening.

According to yet another embodiment of the present invention, the withdrawal string is stitched to the sheet material.

According to further another embodiment of the present invention, the absorbent material is in a bisected state so that the respective halves face each other with the stitched withdrawal string held between the respective halves.

According to an alternative embodiment of the present invention, part of the sheet material is twisted to form the withdrawal string.

According to another alternative embodiment of the present invention, an airflow resistance value of the sheet material is in a range of 0.005 to 0.035 KPa-s/m.

The term "liquid" used herein in reference to the liquid-absorbability and the liquid-permeability means liquid substance such as menstrual blood, urine, physiological salt solution, distilled water, tap water, artificial menstrual blood or artificial urine. In the present invention, composition of artificial menstrual blood and artificial urine is not particularly specified.

### {Advantageous Effects of Invention}

In the catamenial tampon according to the present invention, the tampon body is composed of the absorbent material formed of sponge and the sheet material wrapping the absorbent material. The withdrawal string is attached to the sheet material. The withdrawal string may be pulled to draw out the sponge absorbent material together with the sheet material. In this way, the tampon having been inserted into the vaginal cavity may be reliably drawn out from the vaginal cavity.

### {Brief Description of Drawings}

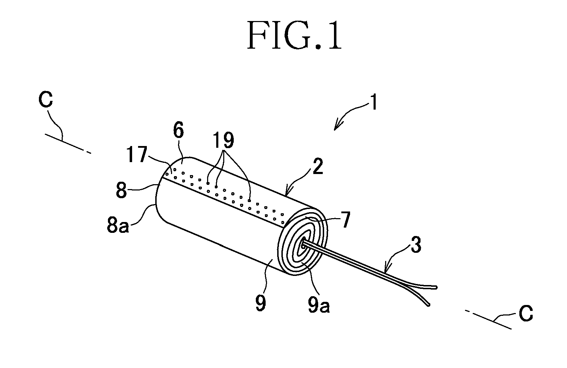

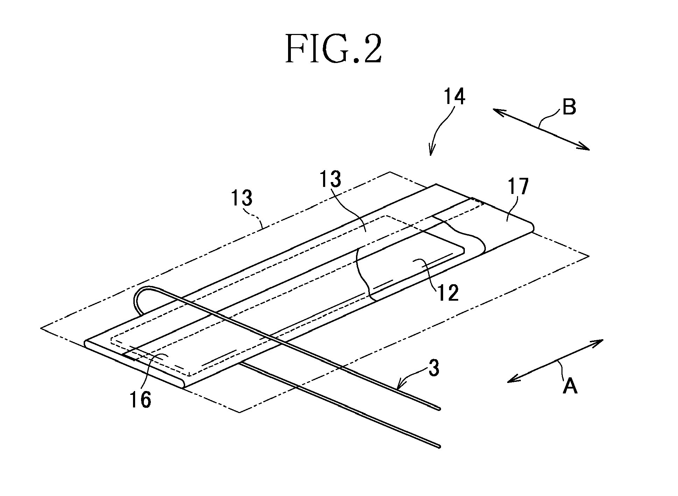

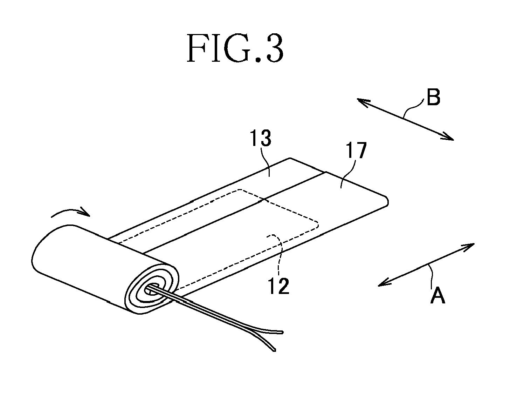

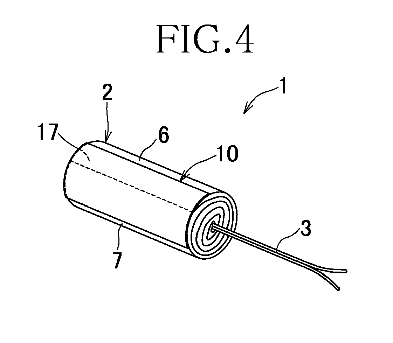

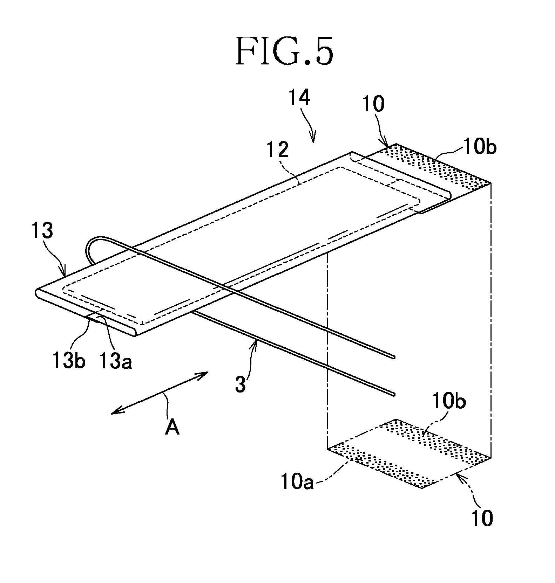

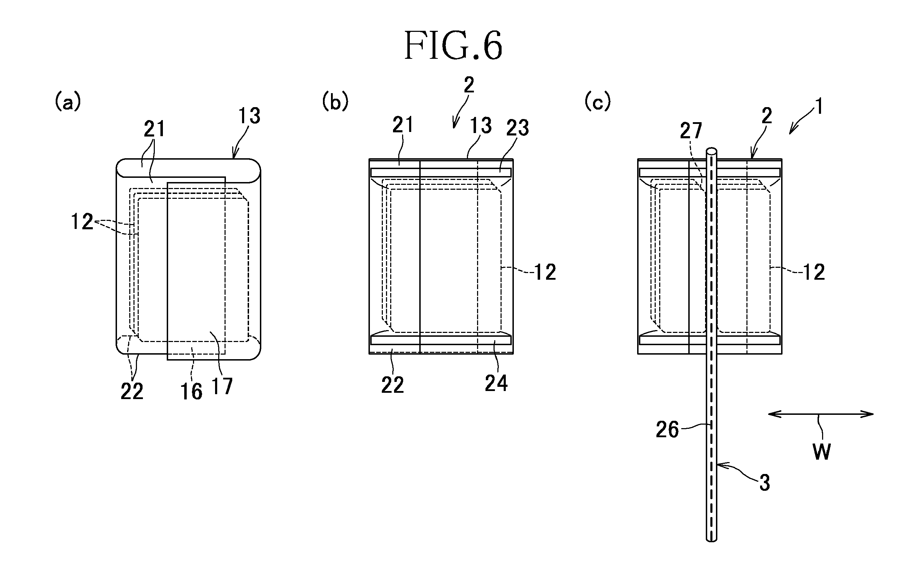

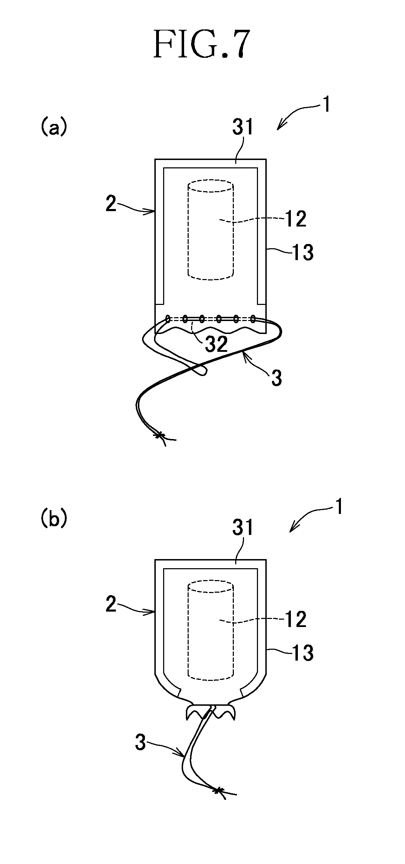

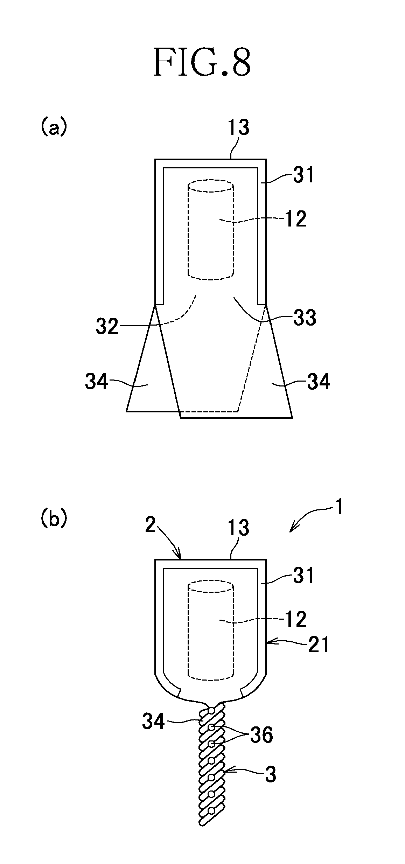

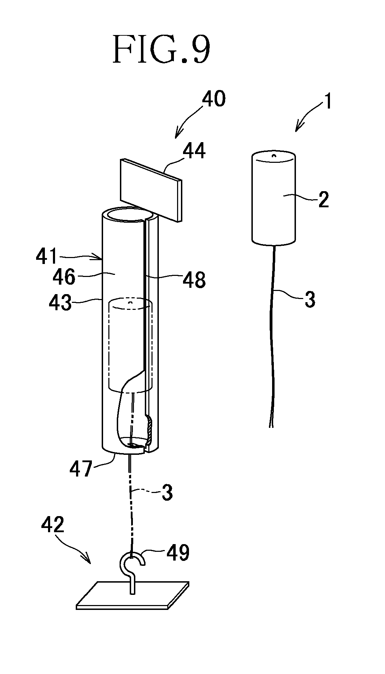

{Fig. 1} Fig. 1 is a diagram illustrating a catamenial tampon according to the present invention.
{Fig. 2} Fig. 2 is a diagram partially illustrating the steps of producing the catamenial tampon illustrated in Fig. 1.
{Fig. 3} Fig. 3 is a diagram similar to Fig. 2.
{Fig. 4} Fig. 4 is a diagram similar to Fig. 1, illustrating another embodiment of the present invention.
{Fig. 5} Fig. 5 is a diagram partially illustrating steps of producing the catamenial tampon according to the embodiment illustrated in Fig. 4.
{Fig. 6} Fig. 6 is a diagram illustrating steps (a), (b) and (c) of producing the catamenial tampon.
{Fig. 7} Figs. 7 (a) and 7 (b) are a diagram illustrating states of sheet material in the course of producing a catamenial tampon different from the catamenial tampon according to the embodiment illustrated in Fig. 7.
{Fig. 8} Fig. 8 is a diagram illustrating steps (a) and (b) of producing a catamenial tampon different from the catamenial tampon according to the embodiment illustrated in Fig. 7.
{Fig. 9} Fig. 9 is a perspective view of a testing jig.

### {Description of Embodiments}

Details of the catamenial tampon according to the present invention will be described hereunder with reference to the accompanying drawings.

Fig. 1 is a perspective view of a catamenial tampon 1 according to the present invention. The catamenial tampon 1 includes a cylindrical tampon body 2 and a withdrawal string 3. The tampon body 2 is roll-shaped so as to have spiral-patterned end surfaces 8a, 9a at a insertion end 8 and a withdrawal end 9. The outer end 17 of the outer layer 6 in the tampon body 2 is permanently joined to the adjacent inner layer 7 in a plurality of spots 19. The withdrawal string 3 extends from a diametrically central portion of the tampon body 2 in an axial direction C of the roll-shaped tampon body 2. This axial direction C corresponds to a direction in which the tampon body 2 is drawn out from the vaginal cavity (not shown) in which the tampon body has been inserted.

Figs. 2 and 3 are diagrams illustrating the steps in a method of producing the catamenial tampon 1. Referring to Fig. 2, a rectangular planar absorbent material 12 formed of a liquid-absorptive sponge is wrapped with a liquid-permeable wrapping material 13 to form an absorbent composite material 14 composed of the absorbent material 12 and a wrapping material 13. The absorbent composite material 14 has an inner end 16 and an outer end 17 opposed to each other in a length direction A and, in the proximity of the inner end 16, the withdrawal string 3 is put on the outer surface of the wrapping material 13 so as to extend about the absorbent composite material 14 in a transverse direction B which is orthogonal to the length direction A. The transverse direction B corresponds to the axial direction C in Fig. 1. Referring then to Fig. 3, the absorbent composite material 14 is rolled up from the inner end 16 toward the outer end 17 so that the tampon body 2 may have both of the spiral-patterned end surfaces 8a, 9a. After having been completely rolled up, the exposed end portion 17 is joined to the adjacent inner layer 7 (See Fig. 1) by the spots 19. To join the outer layer 6 and the adjacent inner layer 7, various joining techniques may be used. For example, the wrapping material 13 may be heat-embossed/debossed to join the outer layer 6 and the adjacent inner layer 7 together.

In the catamenial tampon 1 illustrated in Fig. 1 formed in this manner, the tampon body 2 rolled up from the planar absorbent material 12, which is preferably a sponge, absorbs body exudates and intercepts the flow of the body exudates from the vaginal cavity into which the tampon body 2 has been inserted. The withdrawal string 3 extends outward from the vaginal cavity and this withdrawal string 3 may be pulled to draw out the tampon body 2 from the vaginal cavity. Usually the absorbent material 12 is formed of liquid-absorptive sponges and the type of material from which the sponges are formed is not specified by the present invention. In other words, for example, a sponge described in JP 1991-109067 A, a hydrophilic foam described in JP 2007-105217 A, and a sponge described in JP 2007-197649 A may be used for the present invention. In addition, the liquid-absorptive foam made from, for example, polyurethane may be also used as the sponge in the present invention. These various types of sponges preferably have a hole diameter in a range of about 5 to about 200 µm and a density in a range of about 0.01 to about 0.1 g/m³. The hole diameter may be measured by observing a cross-sectional surface of the sponge at a magnification in a range of x20 to x50 with the use of a scanning electron microscope. The density may be measured on the basis of dimensions of the sponge under no pressure applied thereto. Although these types of sponge are not superior in mechanical strength such as a tensile strength and a tearing strength, the sponge is used in a state wrapped with the wrapping material 13 and the withdrawal string 3 is attached to the wrapping material 13 according to the present invention. In this way, a force to pull the withdrawal string 3 should not act directly upon the sponge and should not damage the sponge.

As material of the wrapping material 13 having the function effect as described above, various types of sheet materials such as a nonwoven fabric formed of thermoplastic synthetic fibers, a perforated plastic film formed of a thermoplastic synthetic resin or a net formed of a thermoplastic synthetic resin may be used. As for the nonwoven fabric, a spunbond nonwoven fabric or a spunlace nonwoven fabric formed of thermoplastic synthetic fibers having a fineness in a range of 0.1 to 5 dtex and a mass per unit area in a range of 8 to 35 g/m² may be used preferably in a hydrophilized state. While both the thermoplastic synthetic fibers of filament type and the thermoplastic synthetic fibers of staple type are useful as the component fibers of the nonwoven fabric, the nonwoven fabric formed of the filament type component fibers is preferable to inhibit fuzzing and/or falling off of the component fibers from the wrapping material 13. Using the filament type fibers, the number of distal end portions of the thermoplastic synthetic fibers existing in the wrapping material 13 may be decreased compared to the case in which the staple type fibers are adopted as the component fibers of the wrapping material 13, whereby these distal endportions should not irritate the skin of a user. When the outer layer 6 and the adjacent inner layer 7 are heat-embossed/debossed in the spots 19 (See Fig. 1), using a core-in-sheath type conjugate fiber composed of the core formed of polyethylene and the sheath formed of polyester, nylon or polypropylene as the thermoplastic synthetic fiber, the heat-embossing/debossing may be finished at a relatively low temperature in a short amount of time. Under external force such as tensile force, bending force or twisting force, the sponge is rather liable to be partially broken into particles and, in view of this, preferably the wrapping material 13 is capable of preventing such particles of the sponge from staying behind within the vaginal cavity after the catamenial tampon has been drawn out from the vaginal cavity. To this end, preferably sheet materials such as a nonwoven fabric, a perforated plastic film or a net each having an airflow resistance value in a range of 0.005 to 0.035 KPa-s/m is used as the wrapping material 13. When the wrapping material 13 having an airflow resistance lower than 0.005 KPa-s/m is used, the particles of the sponge might pass through the wrapping material 13 and, in contrast, when the wrapping material 13 having an airflow resistance higher than 0.035 KPa-s/m, a longer time might be required for menstrual blood to permeate through the wrapping material 13 into the sponge. The airflow resistance values referred to here are values measured by Air Permeability Tester KES-F8-API manufactured by KATOH TECH CO. LTD. or the tester equivalent thereto. In this regard, the wrapping material 13 is preferably stretchable or elastically stretchable and contractible so as not to constrain movement of the tampon body 2 when it swells upon absorption of body exudates.

A tensile strength of the withdrawal string 3 and an attachment strength between the withdrawal string 3 and the wrapping material 13 in the catamenial tampon 1 must be sufficiently high to assure that the tampon body 2, in a state swollen due to the absorption of the body exudates, may be reliably drawn out from the vaginal cavity. An overall strength is preferably 30 N or higher according to a test method described later (See Fig. 9). To meet such a requirement, a withdrawal string 3 composed of five (5) to ten (10) polyester yarns (each being of cotton count 20) twined together may be used. In this regard, a fineness of the individual polyester yarn of cotton count 20 is 29.527 TEX in one example, so a fineness of the withdrawal string composed of, for example, eight polyester yarns is 29.527 TEX x 8 = 236 TEX.

Figs. 4 and 5 are diagrams similar to Figs. 1 and 2, illustrating an embodiment of the present invention. In the catamenial tampon 1 illustrated in Fig. 4, the outer end portion 17 of the outer layer 6 in the tampon body 2 is joined to the adjacent inner layer 7 through a sheet strip 10 used for sealing. The sheet strip 10 is formed of, for example, a nonwoven fabric or a perforated film of a thermoplastic synthetic resin and is liquid-permeable. The withdrawal string 3 is attached to the tampon body 2 in the same manner as in the case of the catamenial tampon 1 illustrated in Fig. 1. Referring to Fig. 5, the absorbent material 12 is wrapped with the wrapping material 13. Side edges 13a, 13b of the wrapping material 13 extending in the longitudinal direction A of the absorbent material 12 overlap with each other. The sheet strip 10 for sealing before this sheet strip 10 covers the absorbent material 12 is indicated by imaginary lines and has both end portions in the longitudinal direction A coated with adhesive so as to define a first sealing region 10a and a second sealing region 10b. In the absorbent composite material 14 composed of the absorbent material 12 and the wrapping material 13, the sheet strip 10 is pressed upward as viewed in Fig. 5 against the edge portions 13a, 13b overlapping with each other, whereby these edge portions 13a, 13b are integrated together with no more possibility of being separated from each other. After having been provided with the withdrawal string 3, the absorbent composite material 14 is spirally rolled up in the same manner as in the case of the absorbent composite material 14 in Fig. 2 to form the roll-shaped tampon body 2. The second sealing region 10b of the sheet strip 10 may be pressed against the inner layer 7 (See Fig. 4) of the tampon body 2 to obtain the catamenial tampon 1 illustrated in Fig. 4. In this way, using the sheet strip 10, both edges 13a, 13b of the wrapping material 13 may be maintained as integral in the course of producing the catamenial tampon 1, whereby the process of producing the catamenial tampon 1 continues smoothly.

The catamenial tampons 1 exemplified in Figs. 1 and 4, respectively, are suitable for the case in which elongate cylindrical tampons are defined by a diameter vs. length ratio in a range of 1 : 2 to 1 : 10. In this regard, the terms "diameter" and "length" mean the dimensions measured on the spirally rolled-up absorbent material 12.

Fig. 6 sequentially illustrates, by (a), (b) and (c), a method of producing a catamenial tampon 1 differing from the catamenial tampon 1 illustrated in Fig. 1. In a step corresponding to Fig. 6 (a), after two or more absorbent material components 12 formed of a liquid-absorptive sponge have been layered together to form the absorbent material 12, this absorbent material 12 is wrapped with the wrapping material 13 formed of a nonwoven fabric. Of the wrapping material 13, an inner end portion 16 overlaps with an outer end portion 17 and a side edge portion 21 as well as another side edge portion 22 overlap with themselves, respectively. In a step corresponding to Fig. 6 (b), the edge portion 21 overlapping with itself and the edge portion 22 overlapping with itself are bonded to themselves, respectively, in associated spots 23, 24 to form the tampon body 2. In a step corresponding to Fig. 6 (c), in a middle region in a width direction W of the tampon body 2, a relatively wide withdrawal string 3 is attached to the outer surface of the tampon body 2 to form the catamenial tampon 1. The withdrawal string 3 is stitched to the tampon body 2 with the use of sewing thread 26. Referring again to Fig. 6 (c), the absorbent material 12 formed of a sponge generally poor in mechanical strength such as a tearing strength is cut off into two parts in the width direction W under action of a sewing needle (not shown) and the sewing thread 26 in the course of stitching the withdrawal string 3 to the absorbent material 12. For the absorbent material 12 cut off into two, it is possible to absorb body exudates through the cut surfaces. In this regard, it is not essential that the catamenial tampon 1 undergoing the steps (a), (b) and (c) of Fig. 6 is cut off in two parts as illustrated in Fig. 6 (c) and the catamenial tampon 1 according to this embodiment will effectively function even when the absorbent material 12 is not cut off into two parts. According to this embodiment, it is possible, for example, to use a withdrawal string 3 composed of six (6) polyester yarns of cotton count 10 and eight (8) cotton yarns of cotton count 10 twined together so as to have a fineness of about 827 TEX and a tensile strength of 120 N, and a sewing thread 26 composed of three (3) cotton threads of cotton count 40 twined together so as to have a tensile strength of 9 N.

Fig. 7 is a diagram illustrating the catamenial tampon 1 according to still another embodiment of the present invention. In the catamenial tampon 1 illustrated herein, the tampon body 2 is composed of a cylindrical absorbent material 12 formed of liquid-absorptive sponge and a sac-like wrapping material 13 formed of a liquid-permeable nonwoven fabric. The sac-like wrapping material 13 is formed of a single sheet of a nonwoven fabric being doubled up and portions thereof facing each other are sealed up or sewed up in a region 31. The wrapping material 13 has an opening 32 of which a periphery is provided with the withdrawal string 3 serving also as a closure withdrawal string. In the state of the catamenial tampon 1 illustrated in Fig. 7 (a), the withdrawal string 3 may be pulled against the wrapping material 13 to bring the catamenial tampon 1 to the state illustrated in Fig. 7 (b) in which the opening 32 is kept closed. Upon insertion of the catamenial tampon 1 in the state as illustrated in Fig. 7 (b) into the vaginal cavity, the withdrawal string 3 extends in the direction in which the tampon body 2 is drawn out. Thus, the tampon body 2 can be drawn out from the vaginal cavity merely by pulling the withdrawal string 3. A relative dimension of the absorbent material 12 and the wrapping material 13 is not limited to that as illustrated and may be appropriately varied so that the catamenial tampon 1 may be easy to use.

For the withdrawal string 3 according to the present invention, a strength at a level of 30 N or higher as measured by the test method exemplified in Fig. 9 is preferably required. This is the strength assuring that the catamenial tampon 1 may be drawn out from the vaginal cavity without breaking the withdrawal string 3 and without detaching the withdrawal string 3 from the tampon body 2. When the withdrawal string 3 is provided separately from the liquid-permeable sheet material adapted to wrap the absorbent material 12 as exemplified in Figs. 4, 6 and 7, a fineness of the individual withdrawal string 3 is preferably in a range of 150 to 1000 TEX. As the withdrawal string 3 illustrated in Figs. 1, 3 and 7, for example, a withdrawal string having a fineness of 210 TEX and composed of two polyester yarns each having a fineness of 105 TEX twined with each other may be used. When the withdrawal string 3 is stitched to the tampon body 2 as the withdrawal string 3 illustrated in Fig. 6, it may be required to increase the width of the withdrawal string 3 to avoid stitch skipping of the sewing yarn. To meet this requirement, two or more relatively fine yarns may be twined or interknitted together to obtain the apparently wider withdrawal string 3 in a range of 50 to 75 TEX, for example. In this regard, the types of yarn used to obtain the withdrawal string 3 are not particularly specified to implement the present invention. For example, a spun yarn of cotton or polyester, a spun yarn of cotton or polyester, a mixed yarn of cotton and polyester or core yarn composed of polyester filament core threads wrapped with core may be used.

Fig. 8 is a diagram illustrating the catamenial tampon 1 according to yet another embodiment of the present invention. In the catamenial tampon 1 illustrated in Fig. 8 (b), the tampon body 2 is composed of the absorbent material 12 formed of a liquid-absorptive sponge and the sac-like wrapping material 13 formed of a liquid-permeable nonwoven fabric. As illustrated in Fig. 8 (a), the liquid-permeable nonwoven fabric is doubled up and the two halves thereof are bonded to each other in the region 31 to obtain the sac-like wrapping material 13. The wrapping material 13 includes the opening 32 and a pair of extended portions 34 extending downward as viewed in Fig. 8 (a) from a peripheral portion 33 of the opening 32. As illustrated in Fig. 8 (b), these extended portions 34 are overlapped with each other and twisted together to form the withdrawal string 3. In the withdrawal string 3 obtained in this manner, these two extended portions of the nonwoven fabric are heat-sealed together at a plurality of spots 36 along the withdrawal string 3. Such a withdrawal string 3 also is formed so as to have, preferably, a strength of 30 N or higher.

Fig. 9 is a perspective view of a jig 40 used to measure tensile strength of the withdrawal string 3 and attachment strength between the withdrawal string 3 and the tampon body 2 in the catamenial tampon 1 exemplified in Fig. 1. The jig 40 has a tampon body holder 41 and a withdrawal string holder 42. The tampon body holder 41 includes a cylindrical portion 43 and a plate-like portion 44 attached to a top of the cylindrical portion 43. The cylindrical portion 43 is a metallic cylinder having an inner diameter of 26 mm and is formed in a peripheral wall 46 and a bottom 47 with a slit 48 having a width of 5 mm. In the bottom 47, the slit 48 extends to a central portion. The withdrawal string 3 is guided through the slit 48 into the cylindrical portion 43 so that the lower end of the withdrawal string 3 may extend downward beyond the bottom 47. The tampon body 2 is inserted from above into the cylindrical portion 43. The plate-like portion 44 of the tampon body holder 41 is held by an upper side chuck. The withdrawal string holder 42 is joined to a surface plate of a tensile tester so that the withdrawal string holder 42 may be located just below the tampon body holder 41. The lower end portion of the withdrawal string 3 vertically extending from the tampon body holder 41 is joined to a hook 49 of the withdrawal string holder 42.

In the tensile tester having the jig 40 and the catamenial tampon 1 set in this manner, the upper chuck holding the plate-like portion 44 is moved upward at a rate of 200 +/- 25 mm/min and the strength (unit: N) at a moment when the withdrawal string 3 is broken or separated from the tampon body 2 is measured. Such strength value is measured on ten samples of catamenial tampon 1 and an average value is determined as the strength of the catamenial tampon 1.

Such strength measurement is conducted on the samples of the catamenial tampon 1 having been subjected to conditioning at a temperature of 20 +/- 2°C and a relative humidity of 65 +/-5 % for 12 hours or longer.

The shape of the tampon body holder 41 may be appropriately varied so as to be compatible with a shape of the tampon body 2 to be strength-measured.

Using the same jig as exemplified, it is also possible to measure tensile strength of the withdrawal string of the catamenial tampon 1 in wet condition. As the tensile tester, for example, AUTOGRAPH-AG-1kNI manufactured by Shimadzu Corporation in Japan may be used.

### {Reference Signs List}

- 1: catamenial tampon
- 2: tampon body
- 3: withdrawal string
- 12: absorbent material

## Claims

1. A catamenial tampon including a tampon body adapted to be inserted into a vaginal cavity and a withdrawal string used to draw out the tampon body from the vaginal cavity, wherein:
the tampon includes an absorbent material formed from a liquid-absorptive sponge and a wrapping material formed of a liquid-permeable sheet material and adapted to wrap the absorbent material; and
the withdrawal string is attached to the sheet material so as to extend in a direction in which the tampon body is drawn out from the vaginal cavity.

2. The catamenial tampon according to Claim 1, wherein the sheet material may be selected from one of a nonwoven fabric formed of thermoplastic synthetic fibers, a perforated film formed of a thermoplastic synthetic resin and a net formed of a thermoplastic synthetic resin.

3. The catamenial tampon according to Claim 1 or 2, wherein the absorbent material is formed of planar absorbent sponge and the withdrawal string is doubled up so as to pinch the planar absorbent sponge in a thickness direction thereof; and
the sponge is spirally rolled up together with the sheet material to form a roll so that the withdrawal string may extend from a diametrical center of the roll in an axial direction of the roll.

4. The catamenial tampon according to any one of Claims 1 through 3, wherein the absorbent material is formed of a columnar sponge and contained within a sac formed of the sheet material; and the withdrawal string is attached to a periphery of an opening defined by the sac so that the withdrawal string may be pulled against the sac to close the opening.

5. The catamenial tampon according to any one of Claims 1 through 3, wherein the withdrawal string is stitched to the sheet material.

6. The catamenial tampon according to Claim 5, wherein the absorbent material is in a bisected state so that the respective halves face each other with the stitched withdrawal string held between the respective halves.

7. The catamenial tampon according to Claim 1 or 2, wherein part of the sheet material is twisted to form the withdrawal string.

8. The catamenial tampon according to any one of Claims 1 through 7, wherein an airflow resistance value of the sheet material is in a range of 0.005 to 0.035 KPa-s/m.
